# EUROPEAN PATENT APPLICATION

(11) **EP 1 158 044 A1**
(43) Date of publication of application: **28.11.2001**
(21) Application number: 00902056.1
(22) Date of filing: 02.02.2000
(51) Int. Cl.: C12N 5/00, C12N 15/11, A61K 38/22, A61P 43/00, A61K 45/00

(54) **METHOD FOR INDUCING DIFFERENTIATION INTO ADIPOCYTES, COMPOUND REGULATING DIFFERENTIATION INTO ADIPOCYTES AND METHOD FOR SCREENING THE SAME**

(30) Priority: 02.02.1999 JP 2462599
(71) Applicant: Helix Research Institute, Kisarazu-shi, Chiba 292-0812 (JP)
(72) Inventor: WAKAO, Rika, Kisarazu-shi, Chiba 292-0814 (JP); WAKAO, Hiroshi, Kisarazu-shi, Chiba 292-0814 (JP)
(74) Representative: VOSSIUS & PARTNER
(86) International application number: JP0000567
(87) International publication number: WO0046348

(57) **Abstract**

Prolactin, one of the components of fetal bovine serum (FBS), was found to have the ability to induce expression of the C/EBPβ gene and PPARγ gene in multipotential mesenchymal stem cells and to induce the differentiation of said cells into adipocytes. In addition, it was revealed that the use of the adipocyte differentiation system using prolactin would enable the screening for compounds that control differentiation of adipocytes.

## Description

### Technical Field

This invention relates to methods for inducing the differentiation of multipotential mesenchymal stem cells into adipocytes, compounds that promote or inhibit such differentiation, as well as methods of screening for same.

### Background Art

Adipocytes play an important role in regulating energy metabolism in the body. Abnormalities in adipocytes are known to be involved in disorders such as obesity and diabetes. Adipocytes arise from a group of cells called multipotential mesenchymal stem cells.

The differentiation from multipotential mesenchymal stem cell into adipocytes is accompanied by alterations in gene expression, the cessation of cell proliferation, and morphologic changes, (O.A. MacDougald and M.D. Lane (1995) Annu. Rev. Biochem. 64:345-373; L. Fajas et al. (1998) Curr. Opin. Cell Biol. 10:165-173). Studies using preadipocytes, such as the 3T3-L1 and 3T3-F422A, revealed several genes important in adipocyte differentiation. Examples of these genes are transcription factors, such as the C/EBP protein family and ADD-1, and nuclear hormone receptors, such as PPARγ, and so on. They are expressed at certain periods during adipocyte differentiation to control the differentiation by promoting the expression of adipocyte-specific genes.

Proteins of the C/EBP family share a common structure, particularly the leucine zipper for dimerization and the basic residue for DNA binding at the C-terminus. Those among the C/EBP family, such as C/EBPα, -β, -δ and CHOP (Gadd153), are known to be involved in adipogenesis (F.T. Lin and M.D. Lane (1992) Genes Dev. 6:533-544; S.O. Freytag et al., (1994) Genes Dev. 8:1654-1663; F.T. Lin and M.D. Lane (1994) Proc. Natl. Acad. Sci. USA 91:8757-8761; W.C. Yeh et al. (1995) Genes Dev. 9:16.8-181; N. Batchvarova et al. (1995) EMBO J. 14:4654-4661). The expression of C/EBPβ mRNA and -δ mRNA is induced at an early stage of 3T3-L1 cell differentiation, whereas the C/EBPα expression is induced at a later stage of the differentiation process (Z. Cao et al. (1991) Genes Dev. 5: 1538-1552). The expression of PPARγ and ADD-1 increases with the progression of cell differentiation (Tontonoz et al., 1994, Cell, 79 1147-1156; J.B. Kim and B.M. Spiegelman (1996) Genes Dev. 10: 1096-1107). Differentiation of preadipocyte or multipotential mesenchymal stem cells into adipocytes occurs with ectopic expression of one of the proteins above belonging to the C/EBP family; therefore, members of the C/EBP families are indicated to be crucial regulation factors for differentiation into adipocytes. For example, conversion into adipocytes is triggered when C/EBPβ is expressed ectopically in multipotential mesenchymal stem cells. The same effect is observed when C/EBPδ is expressed ectopically, albeit with a lower activity (Yeh et al. (1995) Genes Dev. 9: 168-181). Furthermore, when the dominant negative form of C/EBPβ is overexpressed, inhibition of the 3T3-L1 cell differentiation is observed (Yeh et al. (1995) Genes Dev. 9:168-181). C/EBPα is also known to play an important role in adipocyte differentiation, and introduction of an antisense RNA of C/EBPα into the 3T3-L1 cells results in the blockage of differentiation (F.T. Lin and M.D. Lane (1992) Genes Dev. 6:533-544). On the contrary, overexpression of C/EBPα in multipotential mesenchymal stem cells causes adipocyte differentiation, even in the absence of adipogenic hormones (S.O. Freytag et al., (1994) Genes Dev. 8:1654-1663; F.T. Lin and M.D. Lane (1994) Proc. Natl. Acad. Sci. USA 91:8757-8761). The CHOP (Gadd153) protein forms strong dimers with C/EBP α and C/EBPβ to inhibit binding of the C/EBP protein to the DNA (D. Ron and J.F. Habener (1992) Genes Dev. 6:439-453). This results in inhibition of 3T3-L1 cell differentiation into adipocytes (N. Batchvarova et al. (1995) EMBO J. 14: 4654-4661).

The C/EBPα deficient mouse shows a significant decrease in brown adipose tissue (BAT) and white adipose tissue (WAT) (N.D. Wang et al. (1995) Science 269:1108-1112). Further, deficiency of either the C/EBPβ or -δ gene in mouse results in partial inhibition of adipocyte differentiation of primary embryonic fibroblasts derived thereof, and a slight loss in the volume of epidydimal WAT (T. Tanaka et al. (1997) EMBO J. 16:7432-7443). The C/EBPβ and -δ double knock out mouse, however, displays a marked inhibition in adipocyte differentiation and a severe decrease in WAT volume due to the decrease in adipocyte cell numbers (T. Tanaka et al. (1997) EMBO J. 16:7432-7443).

PPARγ is a transcription factor belonging to the ligand-stimulated nuclear hormone receptor super family (S.A. Kliewer et al. (1994) Proc. Natl. Acad. Sci. USA 91:7355-7359; D.J. Mangelsdorf et al. (1995) Cell 83:835-839). This gene is highly expressed in adipose tissue, though expression in other cells is also observed. Enforced expression of the PPARγ in multipotential mesenchymal stem cells induces adipocyte differentiation in the presence of PPARγ ligands/antagonists, namely thiazolidinedione or prostaglandin (Tontonoz et al., (1994) Cell, 79:1147-1156; B.M. Forman et al. (1995) Cell 83:803-812; S.A. Kliewer et al. (1995) Cell 83:813-819). In the adipocytes, PPARγ activates genes, such as 422/aP2, phosphoenol pyrvatecarboxykinase (PEPCK) and lipoprotein lipase (LPL), which are involved in adipocyte differentiation. In fact, promoters of these genes contain PPARγ binding sites (Tontonoz et al., (1994) Genes and Dev. 8:1224-1234; K. Schoonjans et al. (1996) EMBO J. 15:5336-5348). From these experiments described above, it has been proven that the PPARγ is a key gene for adipogenesis. Recently, PPARγ has also been shown to be involved in the regulation of inflammatory reaction and macrophage differentiation (M. Ricote et al. (1998) Nature 391:79-82; C. Jiang et al. (1998) Nature 391:82-86; L. Nagy et al. (1998) Cell 93:229-240; P. Tontonoz et al. (1998) Cell 93:241-252).

ADD-1 is a transcription factor that belongs to another family, which contains a basic helix-loop-helix motif (P. Tontonoz et al. (1993) Mol. Cell. Biol. 13:4753-4759). The expression pattern of ADD-1 during differentiation of 3T3-L1, is similar to that of the PPARγ. As is the case with PPARγ expression, ectopic overexpression of the ADD-1 gene in multipotential mesenchymal stem cell results in conversion into adipocytes in the presence of a PPARγ activating factor (J.B. Kim and B.M. Spiegelman (1996) Genes Dev. 10:1096-1107). Intriguingly, the human homologue of ADD-1, sterol regulatory element-binding protein (SREBP1), is involved in the regulation of genes related to cholesterol metabolism (C. Yokoyama et al. (1993) Cell 75:187-197).

As mentioned above, although these transcription factors are known to play important roles in adipocyte differentiation, expression of genes and function is not limited to adipocytes. Moreover, little is known about the early events in differentiation, particularly how the differentiation of the precursor cell into adipocytes is carried out .

Differentiation of 3T3-L1, preadipocytes, into adipocytes requires adipogenic hormones, such as fetal bovine serum (FBS), methylisobutylxanthine (MIX), dexamethasone (DEX) and insulin. Among these hormones, MIX is known to induce C/EBPβ, and DEX induces C/EBPδ (Z. Cao et al. (1991) Genes Dev. 5:1538-1552). However, little is known about the regulation of PPARγ gene expression in the early stages of adipogenesis.

Concerning the regulation of the PPARγ mRNA, there is a possibility that the increase of C/EBPβ is important for the expression of PPARγ (Z. Wu et al. (1996) Mol. Cell. Biol. 16:4128-4136; Z. Wu et al. (1995) Genes Dev. 9:2350-2363). However, *in vivo* experiments imply little, if any, dependence of the PPARγ expression on C/EBPβ (T. Tanaka et al. (1997) EMBO J. 16:7432-7443). Therefore, there is a need in the art to elucidate the regulatory mechanisms of the molecules involved in adipocyte differentiation, including the expression induction of PPARγ.

### Disclosure of the invention

An object of the invention is to elucidate the regulatory mechanism of adipocyte differentiation, thereby providing new methods for inducing differentiation of adipocytes, as well as compounds that promote or inhibit adipocyte differentiation and methods of screening for these compounds.

In order to solve the problems above, the inventors first searched for factors inducing the expression of the C/EBP family genes, which are important regulating factors in adipocyte differentiation, and the PPARγ gene, which is presumed to be the key gene of adipocyte differentiation. As a result, the inventors discovered that one component of fetal bovine serum (FBS), prolactin, can induce the transcription of both the C/EBPβ gene and the PPARγ gene, at least in multipotential mesenchymal stem cells.

In addition, the inventors discovered the ability of prolactin to convert multipotential mesenchymal stem cells into adipocytes under a strong permissive incubation conditions, namely under the presence of a PPARγ ligand/agonist.

Moreover, the inventors tested the involvement of the prolactin receptor in the differentiation of multipotential mesenchymal stem cells into adipocytes. Consequently, the inventors uncovered that the prolactin receptor induces differentiation of multipotential mesenchymal stem cells into adipocytes at a high efficiency in the presence of prolactin and other activating factors of PPARγ.

That is, the inventors have succeeded in demonstrating for the first time that signals by both prolactin and PPARγ work cooperatively to induce differentiation of multipotential mesenchymal stem cells into adipocytes.

Furthermore, the inventors established that screening for compounds that control differentiation of adipocyte can be achieved by utilizing the above mentioned adipocyte differentiation system, using prolactin and the factors mentioned above related in the signal transduction of adipocyte differentiation as the target.

Thus, the present invention relates to methods for inducing differentiation of multipotential mesenchymal stem cells into adipocytes, as well as to compounds that promote or inhibit differentiation into adipocytes and methods of screening for those compounds. More specifically, the present invention relates to the following:
(1) A method for differentiating a multipotential mesenchymal stem cell into adipocytes, which includes incubation of the multipotential mesenchymal stem cell in the presence of prolactin or a substance with an equivalent effect;
(2) The method of (1), wherein said incubation is conducted in the presence of a PPARγ activator;
(3) The method of (1) or (2), wherein the multipotential mesenchymal stem cells express the exogenous prolactin receptor;
(4) The method of any of (1) to (3), wherein the multipotential mesenchymal stem cell is the NIH-3T3 cell;
(5) A method of screening for inhibitors or accelerators of adipocyte differentiation, which comprises the steps of:
   (a) incubating multipotential mesenchymal stem cells in the presence of a test compound and prolactin or a substance with an equivalent effect,
   (b) detecting the differentiation of said cells into adipocytes,
   (c) selecting the test compound which inhibits or promotes the differentiation, by comparing the result of (b) with that under the absence of the test compound (control) ;
(6) The method of (5), wherein a PPARγ activator is added with the test compound;
(7) The method of (5) or (6), wherein the multipotential mesenchymal stem cells express the exogenous prolactin receptor;
(8) The method of any of (5) to (7), wherein adipocyte differentiation is detected using as an indicator(a) fat accumulation in the cytoplasm, (b) expression of genes inducing adipocyte differentiation, or (c) expression of adipocyte marker genes;
(9) The method of any of (5) to (8), wherein the multipotential mesenchymal stem cells are NIH-3T3 cells;
(10) A method of screening for inhibitors or accelerators of adipocyte differentiation, which comprises the steps of:
   (a) contacting prolactin with a test compound, and
   (b) selecting the test compound that binds to prolactin;
(11) A method of screening for inhibitors or accelerators of adipocyte differentiation, which comprises the steps of:
   (a) contacting the prolactin receptor and a test compound, and
   (b) selecting the test compound that binds to the prolactin receptor;
(12) A method of screening for inhibitors or accelerators of adipocyte differentiation, which comprises the steps of:
   (a) contacting prolactin with the prolactin receptor in the presence of a test compound, and
   (b) selecting the test compound that inhibits or promotes binding of prolactin to the prolactin receptor;
(13) A method of screening for inhibitors or accelerators of adipocyte differentiation, which comprises the steps of:
   (a) providing cells expressing the endogenous prolactin receptor and transfecting them with a vector containing a promoter, which is activated in response to prolactin, and a reporter gene functionally fused downstream to it,
   (b) contacting the cells with (i) a test compound or (ii) the test compound with prolactin to said cell, and
   (c) detecting the activity of the reporter gene in said cell;
(14) An adipocyte differentiation inhibitor, which contains a prolactin inhibitor as the active ingredient;
(15) An adipocyte differentiation inhibitor, which contains a prolactin receptor inhibitor as the active ingredient;
(16) An adipocyte differentiation accelerator, which contains a prolactin activator as the active ingredient;
(17) An adipocyte differentiation accelerator, which contains a prolactin receptor activator as the active ingredient;
(18) An adipocyte differentiation inhibitor or accelerator, which can be isolated by the methods of (5) to (13);
(19) An adipocyte differentiation accelerator, which contains prolactin as the active ingredient;
(20) A PPARγ expression-inducing agent, which contains prolactin as the active ingredient;
(21) A C/EBPβ expression-inducing agent, which contains prolactin as the active ingredient;
(22) A compound that inhibits or promotes adipocyte differentiationby inhibiting or promoting intracellular signal transduction of prolactin.

In this invention, the term "multipotential mesenchymal stem cell" refers to cells, which exist and function individually (unicellular) in the embryo and have the ability to differentiate into cartilage cells, myoblasts, and adipocytes. Furthermore, the term "adipocyte" herein refers to major cells constituting the adipose tissues, that contain fat inside the cells and that function in lipogenesis and lipolysis of the body energy. And the terms "differentiation into adipocytes", "adipocyte differentiation" and "differentiation of adipocyte" refer not only complete differentiation but also various changes that accompany the induction of adipocyte differentiation.

In the present invention, prolactin was found to have the ability to induce differentiation of multipotential mesenchymal stem cells into adipocytes. Thus, this invention firstly relates to a method for differentiating multipotential mesenchymal stem cells into adipocytes, including the process of incubating multipotential mesenchymal stem cells in the presence of prolactin. In addition to prolactin in its natural form, recombinant prolactins, prepared by gene recombination techniques, and commercially available prolactins (for example, that from Sigma) may be used in this invention. Furthermore, in addition to prolactin in its natural form, mutant forms and partial peptides thereof may further be used so long as they have the ability to induce the differentiation of multipotential mesenchymal stem cells into adipocytes.

In the present invention, it was also found that activation of the prolactin receptor induces differentiation of multipotential mesenchymal stem cells into adipocytes. Thus, it is possible to use a substance with an equivalent function to prolactin, having the ability to activate the prolactin receptor, in the present invention. The term "substance with an equivalent function as prolactin" refers to compounds, which have the ability to activate the prolactin receptor and transduce signals into the cells like prolactins. One such compound is, for example, placental lactogen (Cohick et al., (1996) Mol. Cell. Endocrinol. 116:49-58).

The multipotential mesenchymal stem cell used in this invention are preferably NIH-3T3 cells, but BALA/c3T3 and Swiss3T3 may also be used. As shown in the examples herein, when prolactin is interacted with multipotential mesenchymal stem cells overexpressing the prolactin receptor, rise in the efficiency of differentiation of such multipotential mesenchymal stem cells into adipocytes was observed. Thus, it is preferable to use multipotential mesenchymal stem cells that overexpress the prolactin receptor in the present invention. It is possible to raise the expression levels of the prolactin receptor of the cells, for example, by introducing the prolactin receptor genes exogenously. Specifically, as mentioned in the examples, an expression vector harboring a cDNA encoding the prolactin receptor may be introduced into the cells. The expression level can be appropriately regulated by selecting appropriate promoters, gene copy numbers, and such to be used.

The prolactin receptor gene may be isolated using methods known to those skilled in the art. For example, one can screen the cDNA library derived from cells or tissues (such as liver) that express the prolactin receptor with primers prepared based on the base sequence of the prolactin receptor.

As an expression vector for expressing the gene of interest in the multipotential mesenchymal stem cells, any expression vector that can be expressed in mammalian cells, for example, such as the pME18S vector (Mol. Cell. Biol. 8:466-472(1988)) or a vector harbouring the CMV promoter, can be used. The insertion of the cDNA into the vector may be accomplished by conventional methods, like the ligation method utilizing the restriction enzyme sites (Current protocols in Molecular Biology edit. Ausubel et al. (1987) Publish. John Wiley & Sons,Section 11.4-11.11). Regarding the introduction of the vector into the multipotential mesenchymal stem cell, for example, methods like calcium phosphate transfection, electroporation (Current protocols in Molecular Biology, edit. Ausubel et al. (1987) Publish. John Wiley & Sons, Section 9.1-9.9), lipofectAMINE method (GIBCO-BRL), microinjection methods, and such may be used, as well as the method utilizing the retrovirus (Pear et al., (1993) Proc. Natl.Acad.Sci. USA, 90:8392-8396).

Differentiation of the multipotential mesenchymal stem cells into adipocytes can be done, for example, following the method described in the examples.

The DMEM medium supplemented with calf serum is generally used as the growth medium for the multipotential mesenchymal stem cells. On the other hand, as a differentiation medium, the DMEM medium to which at least one adipocyte differentiation-inducing hormone, such as DEX, MIX, insulin, FBS, and so on, is added may also used.

According to the examples of the present application, it was revealed that the addition of both prolactin and PPARγ activator to the medium significantly increased the differentiation of precursor cells into adipocytes. Thus, the incubation medium for mutipotential mesenchymal stem cells preferably includes a PPARγ activator. Preferably, the PPARγ activator should be added to the culture medium when the PPARγ expression reaches the highest peak (for example, in the example of the present invention, 48 hr after the stimulation for induction of the adipocyte differentiation). Examples of PPARγ activators include thiazolidinedione (such as troglitazone, englitazone, pioglitazone), ETYA, BRL49653, or 15 deoxy-Δ12,14-prostaglandine J2, and so on.

Furthermore, it was discovered herein that the prolactin-prolactin receptor mediated signal transduction is involved in the differentiation of multipotential mesenchymal stem cells into adipocytes. It is expected that one can inhibit or promote the differentiation of adipocytes by inhibiting or promoting the signal transduction mediated by prolactin-prolactin receptor. Thus, this invention also relates to inhibitors or accelerators of adipocyte differentiation that contain as the active ingredient compounds, which inhibit or promote the signal transduction mediated by prolactin-prolactin receptor. The inhibitor or the accelerator of adipocyte differentiation in the present invention comprises, for example, accelerators of adipocyte differentiation which contain as the active ingredient prolactin, a prolactin activator or a prolactin receptor activator, as well as inhibitors of adipocyte differentiation which interferes with the prolactin signal transduction.

These inhibitors or accelerators may be screened, for example, utilizing the above-mentioned system which differentiates multipotential mesenchymal stem cell into adipocyte. That is, the screening can be accomplished by adding a test compound to the system which differentiates multipotential mesenchymal stem cells into adipocytes, as mentioned above, and detecting the differentiation efficiency from multipotential mesenchymal stem cells to adipocytes. More specifically, the screening method of the present invention comprises the following steps: (a) incubating the multipotential mesenchymal stem cells in the presence of (i) a test compound and (ii) prolactin or a substance with an equivalent effect; (b) detecting differentiation of said cells into adipocytes; (c) selecting compounds that inhibit or intensify the changes compared to the changes that occur in the absence of the test compound (control).

The test compounds applied to the screening method include, for example, purified proteins (including antibodies), expressed products of a gene library, synthetic peptide libraries, cell extracts, cell culture supernatants, synthetic low molecular weight compound libraries, ribozymes, antisense nucleic acids, and so on, but is not limited to these materials.

For example, when antibodies are tested as the test compounds, they can be in any form, without limitation. Polyclonal antibodies and monoclonal antibodies, as well as portions having antigen-binding capacity, are included in the present invention. Preparation of antibodies may be accomplished according to conventional methods (Current protocols in Molecular Biology edit. Ausubel et al. (1987) Publish. John Wiley & Sons. Section 11.12-11.13; Current protocols in Molecular Biology edit. Ausubel et al. (1987) Publish. John Wiley & Sons, Section 11.4-11.11; *Functional transplant of megabase human immunoglobulin loci recapitulates human antibody response in mice,* Mendez,M.J et al. (1997) Nat.Genet.15:146-156; Methods in Enzymology 203:99-121(1991)).

When a protein or RNA is used as the test compound, instead of reacting them to the cell directly, they may be expressed inside the cell by inserting a cDNA encoding them into an expression vector, and introducing the vector into the cells following the method described later for the expression of prolactin receptor cDNA.

In detail, for example, this screening method may be carried out in the presence of the tested compound by incubating the multipotential mesenchymal stem cells in a medium containing prolactin, and detecting the differentiation of such cells into adipocytes as described in Examples 1 to 3. Multipotential mesenchymal stem cells overexpressing the prolactin receptor may be used in this method as described in Example 4. Furthermore, evaluation of the function of the test compound, whether the test compound further promotes the differentiation into adipocytes synergistically with prolactin or the prolactin receptor, can be conducted, by reacting the test compound (including the transfer of the cDNA that correspond to the test compound) to the cell. Likewise, verification, whether or not the test compounds inhibits the adipocyte differentiation of multipotential mesenchymal stem cell by prolactin or the prolactin receptor can be performed by the same type of experiment.

The verification of the differentiation of multipotential mesenchymal stem cells into adipocytes in the screening method of this invention may be carried out, for example, by detecting the phenotypic characters of adipocytes, such as the formation of fat droplets, which are accumulations of fat in the cytosols, and morphological changes of the cells (fibrous undifferentiated cells become round shaped). The detection may be performed by observing with a microscope. To determine whether these observed droplets contain fat, the cells may be fixed with formaldehyde and such, and then stained with the Oil-Red-O reagent. According to this staining method, fatty acid, neutral fat, cholesterol ester, and so on are stained dark red, whereas phospholipid and serebroside are stained light red. Also it is possible to validate in a molecular biological manner using as the indicator the expression of adipocyte marker genes or genes that induce the differentiation into adipocytes. For example, aP2, GPD, Adipsin (Cook et al., (1987) Science 237:402-5), leptin (MacDougald et al., (1995) Proc. Natl. Acad. Sci. USA, 92:9034-7), and so on may be mentioned as the adipocyte marker gene. On the other hand, PPARγ, C/EBPβ, C/EBPδ, C/EBPα, ADD-1, and so on may be mentioned as genes inducing differentiation into adipocytes. The expression of these genes may be detected at the transcriptional level by the Northern blot method or the RNA protection method (Maniatis et al., Molecular Cloning, Cold Spring Harbor Laboratory). The translational levels of these genes may be analyzed by the Western blot method or the immunoprecipitation methods.

If significant inhibition of the differentiation into adipocytes is detected by the addition of the test compound during the detection procedure of differentiation of multipotential mesenchymal stem cells into adipocytes, the test compound is determined to be a candidate adipocyte differentiation inhibitor. Whereas, detection of promotion of the differentiation into adipocyte indicates that the test compound is a candidate adipocyte differentiation accelerator. The accelerator of the present invention does not have to convert cells into adipocytes completely, so long as the addition of the compound accelerates the conversion into adipocytes significantly as compared to the absence of the accelerator. Likewise, the inhibitor of the present invention does not have to inhibit conversion of the adipocytes completely, so long as the addition of the compound inhibits conversion into adipocytes significantly as compared to the absence of the compound.

Compounds isolated from the inventive screening method include those which directly interact with prolactin or the prolactin receptor, as well as compounds that function downstream of the prolactin receptor, in the intracellular signal transduction pathway of adipocyte differentiation mediated by the prolactin-prolactin receptor. Examples of compounds that directly interact with prolactin or the prolactin receptor include compounds which react with the prolactin receptor and either inhibit the signal transduction from prolactin or show an equivalent effect as prolactin (agonist or antagonist of the prolactin receptor), and compounds that react with prolactin to either accelerate or inhibit its binding to the prolactin receptor.

The screening of the accelerator or inhibitor of adipocyte differentiation of the present can be conducted using prolactin and/or the prolactin receptor directly as the target. One embodiment of this screening method utilizes prolactin as the target and it includes the following steps: (a) contacting prolactin with a test compound, and (b) selecting the compound that binds to prolactin. Another embodiment of this screening uses the prolactin receptor as the target and includes the following steps: (a) contacting the prolactin receptor with a test compound, and (b) selecting the compound that binds to the prolactin receptor.

Further, another embodiment of the screening uses both prolactin and the prolactin receptor as targets and includes the following steps: (a) contacting prolactin with the prolactin receptor in the presence of a test compound, and (b) selecting the compound that inhibits or promotes the binding of prolactin to its receptor.

The prolactin receptor used in this invention may be a purified protein, a membrane fraction or protein expressed on the cell surface. For example, the screening may be conducted by incubating prolactin receptor-overexpressing multipotential mesenchymal stem cells in the presence of a test compound and prolactin, and detecting the inhibition or promotion of prolactin-prolactin receptor binding using as an indicator of the differentiation into adipocyte.

Furthermore, the screening for an accelerator or inhibitor of adipocyte differentiation of the present invention may be carried out utilizing a reporter system. The method includes the following steps: (a) providing cells expressing the endogenous prolactin receptor and introducing a vector, which contains a promoter that is activated by prolactin and a reporter gene functionally fused downstream to it, (b) contacting either a test compound or a test compound with prolactin to said cell, and (c) detecting the reporter gene activity in said cells.

The promoter activated by prolactin may be the casein promoter (Wakao, H. et al., EMBO J, (1994), 13:2182-2191), but is not limited to this promoter. The reporter gene may be the firefly luciferase (Wakao, H. et al., EMBO J, (1994) 13:2182-2191), but is not limited to this reporter gene. The vector mentioned above, including the reporter gene fused downstream to the promoter, can be constructed by recombinant gene techniques publicly known to those skilled in the art.

Any cell that endogenously expresses the prolactin receptor, for example, CHO (Chinese hamster ovary) cells, may be chosen to transfer the above vector.

In this screening method, if an increase or decrease of the reporter activity is detected, the test compound may be a candidate accelerator or inhibitor of adipocyte differentiation, respectively.

The compound obtained by the screening methods of the present invention may be a candidate for promoting or inhibiting adipocyte differentiation, by regulating the signal transduction mediated by prolactin-prolactin receptor. To determine whether the obtained compound actually regulates adipocyte differentiation, the compounds are applied to the above mentioned differentiation system of multipotential mesenchymal stem cells into adipocytes, and differentiation of multipotential mesenchymal stem cells into adipocytes is detected.

The compounds obtained from the screening method of this invention, having the activity to inhibit or promote adipocyte differentiation, may be used for various medical applications. For example, the differentiation accelerator in combination with an ameliorating drug for insulin resistant diabetes, such as Noscal (thiazolidinedione), would be expected to ameliorate insulin resistance diabetes. Although the molecular mechanisms of thiazolidinedione group drugs to relieve insulin resistance *in vivo* are not revealed completely, it has been reported that the thiazolidinedione group drugs relieve the inhibitory action of TNF- α in 3T3-L cells (Szalkowski D et al. (1995) Endocrinology 136: 1474-1481). Also, it is hypothesized that the thiazolidinedione group drugs promote differentiation of adipose precursor cells into adipocytes, decreasing the number of large adipocytes which trigger the insulin resistance, and increasing the number of benign small adipocytes which promote insulin sensitivity (Okuno A et al. J.Clin. Invest. (1998) 101(6):1354-61).

In the example of the present invention, prolactin was shown to induce expression of the C/EBPβ gene and the PPARγ gene in multipotential mesenchymal stem cells. Thus, the present invention includes PPARγ expression-inducing agents and C/EBPβ expression-inducing agents, which contain prolactin as the active ingredient.

In the case where the above-mentioned compound, which has the ability to control the differentiation of adipocyte, is used as a medicament, besides administering the compound directly to the patients, the compound may be administered after preparing drugs by methods known in galenics. For example, it may be administered as a preparation by combining it with an appropriate pharmacologically acceptable carrier or media, specifically, sterile water or physiological salt solution, vegetable oil, emersion, suspension, and so on. Administration to the patient may be conducted according to methods known to those skilled in the art, for example, arterial injection, intravenous injection, hypodermic injection, and such. On the other hand, in the case where the compound is encoded by a DNA, the DNA may be inserted into a vector for gene therapy, and may be used in gene therapy. The dosage and administration method depend on the body weight, age, condition, and such of the patient but one skilled in the art may select an appropriate dosage and method. Furthermore, above-mentioned compounds, which can control the differentiation of adipocytes, may be used as reagents for various experiments to control the differentiation of adipocytes.

### Brief Description of the Drawings

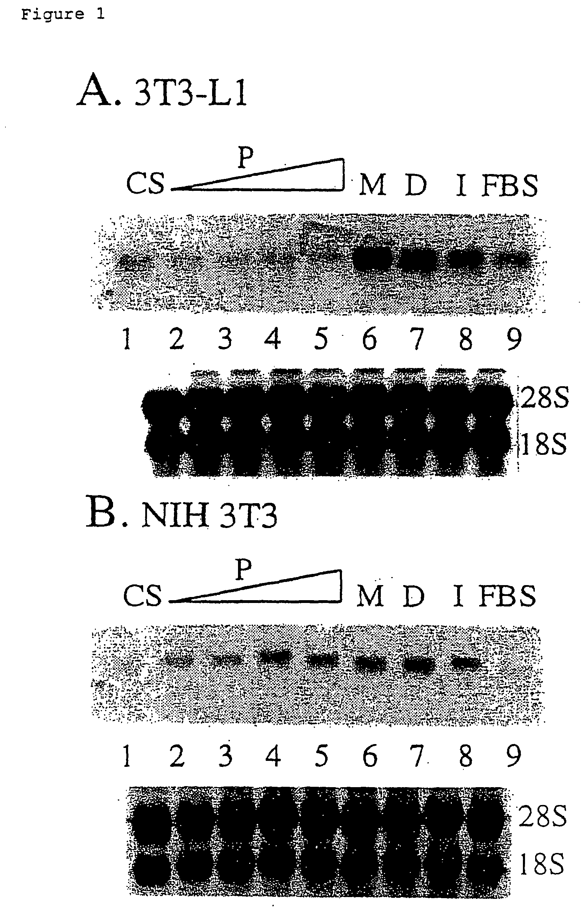

Figure 1 shows the induction of C/EBPâ mRNA in 3T3-L1 cells (A) and NIH-3T3 cells (B) by different effectors.

Cells cultured to confluence were incubated for 3 hours on DMEMs containing each of the below mentioned effectors, and either 10% calf serum (CS) (lanes 1 to 8) or 10% FBS (fetal bovine serum) (lane 9). The total RNA was extracted from the cells and the expression of C/EBPβ was assessed by Northern blot hybridization. Equal amounts (8 µg/lane) of the RNA were loaded on each lane, electrophoresed, and confirmed by ribosomal RNA staining. Figure 1 shows the results with addition of the following effectors respectively: lane 1- without effector; lanes 2 to 5- 37 ng/ml, 111 ng/ml, 333 ng/ml and 1 µg/ml of prolactin, respectively; lane 6 (M)- 0.5 mM MIX; lane 7 (D)- 1 µM DEX; and lane 8 (I)- 10 µg/ml insulin.

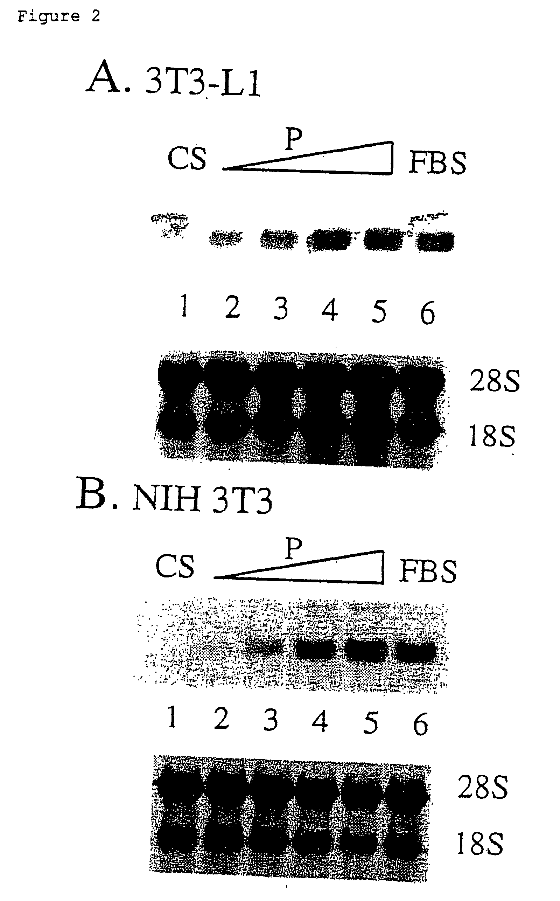

Figure 2 shows the dose-dependent induction of PPARγ mRNA by prolactin in 3T3-L1 cells (A) and NIH-3T3 cells (B).

Cells cultured to confluence were incubated in the DMEM containing 10% calf serum (CS), insulin, DEX and MIX, with increasing amount of prolactin, for 48 hours (lanes 1 to 5). As the control, confluent cells were cultured in the DMEM containing 10% FBS, insulin, DEX and MIX (lane 6). Total RNA was loaded on each lane in equal amounts (4 µg/lane) and electrophoresed and was subjected to Northern blot analyses. The amount of prolactin used in this study was following: lane 1- without prolactin; and lanes 2 to 5- 37 ng/ml, 111 ng/ml, 333 ng/ml; and 1 µg/ml of prolactin, respectively.

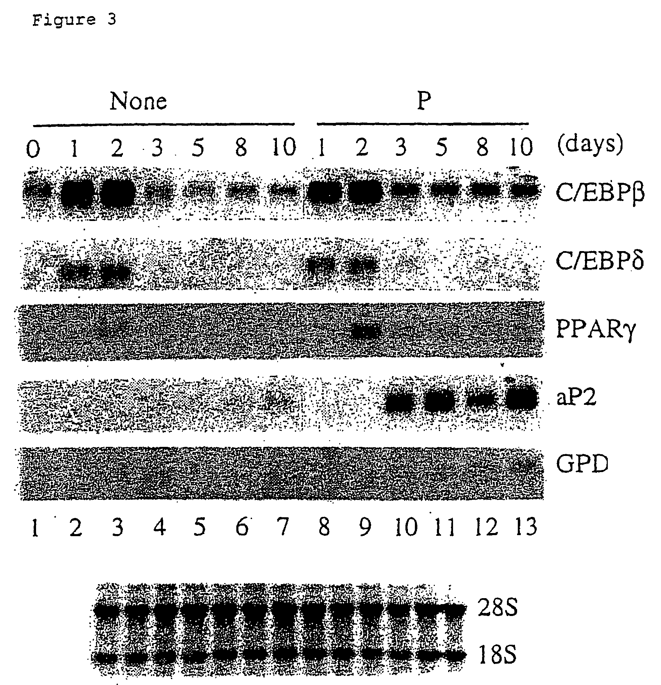

Figure 3 shows the effect of prolactin on the adipocyte differentiation program of the NIH-3T3 cell.

Cells cultured to confluence were incubated in the DMEM containing 10% FBS, insulin, DEX and MIX, with 1 µg/ml of prolactin (P) or without prolactin (None) for 48 hours. Then, cells were incubated in the DMEM containing 5 µM troglitazone, 2.5 µg/ml insulin and 10% FBS, with 1 µg/ml of prolactin (P) or without prolactin (None). After the induction of differentiation, the total RNA was isolated at the indicated time points (days). Equal amounts (3 µg) of the RNA were loaded onto each lane and electrophoresed, and expression of the indicated genes was analyzed by Northern blot hybridization.

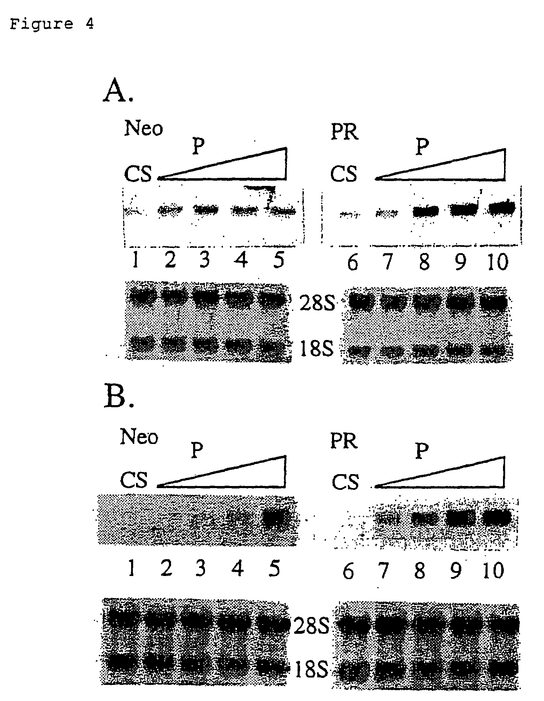

Figure 4 shows the increase in sensitivities of induction of the C/EBPβ mRNA and PPARγ mRNA, due to the ectopic expression of the prolactin receptor in NIH-3T3 cells

The NIT-3T3 cells were transfected with pSV2neo alone (Neo), or with pSV2neo and the expression vector of the prolactin receptor (PR).

(A): Cells cultured to confluence were incubated in the DMEM containing 10% calf serum (CS) with increasing doses of prolactin for 3 hours (lanes 1 to 5, and 6 to 10). The total RNA was isolated. An equal amount (3µg) of the RNA loaded on each lane was electrophoresed, and the C/EBPβ mRNA was analysed by Northern blot hybridization. Each lane shows the result with different prolactin concentrations: lanes 1 and 6- without prolactin; lanes 2 and 7- 37 ng/ml; lanes 3 and 8, 111 ng/ml; lanes 4 and 9- 333 ng/ml; and lanes 5 and 10- 1 µg/ml.

(B): Cells were incubated in the DMEM containing 10% calf serum (CS), insulin, DEX and MIX with increasing amounts of prolactin for 48 hours (lanes 1 to 5 and 6 to 10). The PPARγ mRNA was assessed by Northern blot analysis using 2 µg of the total RNA. The used amounts of prolactin were the same as that in (A).

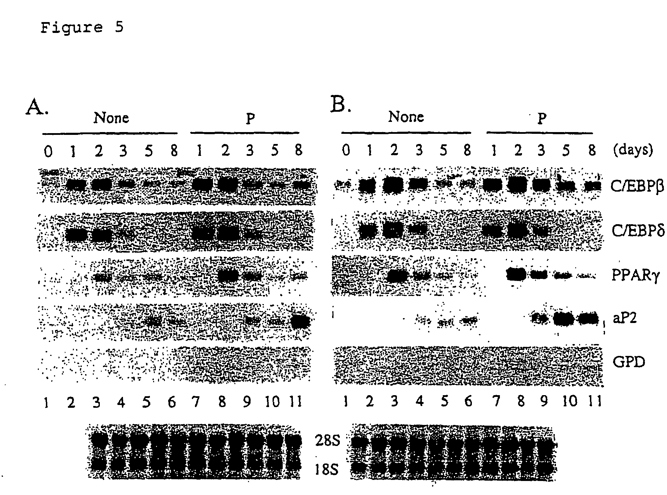

Figure 5 shows the effects of the prolactin receptors on the adipocyte differentiation program of NIH-3T3 cells.

Cells stably transfected with the prolactin receptor were cultured to confluence, and then were incubated in the presence of troglitazone in the FBS differentiation medium without prolactin (None) or with 1 µg/ml prolactin (P). After the induction of differentiation, the total RNA was isolated at indicated time points (days) . An equal amount (3 µg) of the RNA was loaded on each lane, electrophoresed, and then analyzed by Northern blot hybridization for the expressions of adipocyte-specific genes. (A) is the result of the cells that stably expressed the neomycin-resistance gene, and (B) is the result of the cells that stably expressed both the neomycin-resistance gene and the prolactin receptor gene.

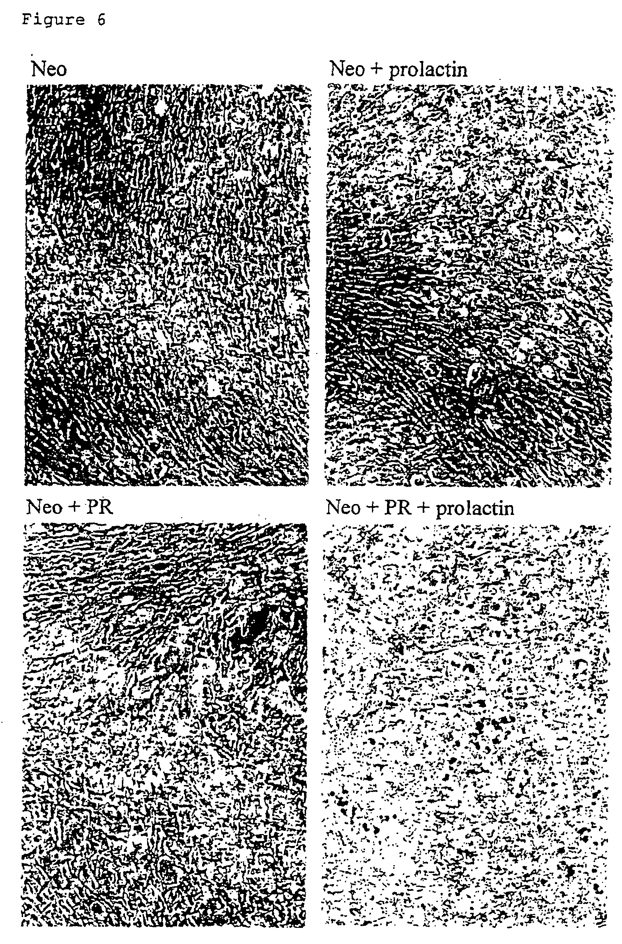

Figure 6 shows the micrographs of the NIH-3T3 cells that expressed the neomycin-resistance gene alone (Neo), or the neomycin-resistance gene and the prolactin receptor gene (Neo+PR).

Cells stably transfected with the above genes were cultured to confluence, and then incubated for 10 days under a high permissive condition, either in the absence or presence of 1 µg/ml prolactin (the latter is indicated as "+prolactin"). The cells were then fixed and stained with Oil-Red-O. Cells expressing the prolactin receptor gene became rounded-shaped and were stained red with Oil-Red-O.

### Best Mode for Carrying out the Invention

The present invention will be specifically explained with reference to the following examples. However, it should be noted that the present invention is not limited by these examples.

### [Reference] Isolation of the cDNA for the preparation of probes for the Northern blot analysis:

To use the cDNAs of C/EBPα, C/EBPβ, C/EBPδ, PPARγ, aP2 and GPD cDNA as probes for the Northern blot analysis, these cDNAs were isolated from the 3T3-L1 adipocyte cDNA library constructed on the 9th day from the induction of differentiation, using following synthetic oligonucleotides that correspond to each gene:

Mouse C/EBPα, 5'-ATGGAGTCGGCCGACTTCTACGAGGCGGAG-3' (SEQ ID
NO: 1)
Mouse C/EBPβ, 5'-ATGCACCGCCTGCTGGCCTGGGACGCAGCA-3'(SEQ ID
NO: 2)
Mouse C/EBPδ, 5'-ATGAGCGCCGCGCTTTTCAGCCTGGACAGC-3'(SEQ ID
NO: 3)
Mouse PPARγ, 5'-ATGGTTGACACAGAGATGCCATTCTGGCCC-3'(SEQ ID
NO: 4)
Mouse aP2, 5'-ATGTGTGATGCCTTTGTGGGAACCTGGAAG-3'(SEQ ID
NO: 5)
Mouse GPD, 5'-ATGGCGTTTCAAAAGGCAGTGAAGGGGACT-3'(SEQ ID
NO: 6)

To isolate each gene, the above synthetic oligonucleotides were DIG-labeled and used for screening the 3T3-L1 adipocyte cDNA library to obtain positive clones. Each clone from the lamda gt 22 phage was transferred to pZL1 plasmid (Gibco BRL).

### [Example 1] Effects of prolactin on expression of the C/EBPβ mRNA in multipotential mesenchymal stem cells (NIH-3T3 cells) and 3T3-L1 adipocyte precursor cells:

The expression of C/EBPβ mRNA is known to be induced by various stimuli, such as MIX, Dex, insulin, LPS, interleukin-1 (IL-1), IL-6, growth hormones, etc. (S. Akira et al. (1990) EMBO J. 9:1897-1906; Z. Cao et al. (1991) Genes Dev. 5:1538-1552; R.W. Clarkson et al. (1995) Mol. Endocrinol. 9:108-120). The inventors of the present invention studied whether prolactin induced C/EBPβ mRNA in 3T3-L1 preadipocytes (National Institute of Health Cell Bank, JCRB9014) and multipotential mesenchymal stem cells (NIH-3T3 cells)(Riken Cell Bank, Catalog No. RCB0150).

Cells cultured to confluence in DMEM (Japan Biological Institute) containing 10% calf serum (CS) (GIBCO BRL) were incubated with different amounts (37 ng/ml, 111 ng/ml, 333 ng/ml and 1 µg/ml) of prolactin (Sigma) for 3 hours, in the presence or absence of various effectors mentioned below. Then, the total RNA was isolated according to the method described in the literature (P. Chomczynski and N. Sacchi, (1987) Anal. Biochem. 162: 156-159), electrophoresed on 1% agarose / 2.2 M formaldehyde gels, and transferred to nylon membranes (T. Maniatis et al., (1989) Molecular Cloning: A Laboratory Manual, Second Edition, Cold Spring Harbor, New York, Cold Spring Harbor Laboratory Press). The rRNA was stained with methylene blue (D.L. Herrin and G.W. Schmidt (1988) Biotechniques 6:196-197, 199-200) to confirm equal RNA transfer.

According to the protocol of Boehringer Mannheim (current Roche Diagnostics) , pZL1 containing the C/EBPβ cDNA was linearized with EcoRI and transcribed in vitro to provide DIG-labeled RNA probes. Using this antisense RNA probes, Northern hybridization was performed according to the attached protocol (Boehringer Mannheim (current Roche Diagnostics)).

The results are shown in Figure 1A and 1B. No prolactin-dependent C/EBP β mRNA expression was observed in the 3T3-L1 cells (Figure 1A, lanes 1 to 5). However, as reported previously, MIX (0.5 mM) (lane 6), DEX (1 µM) (lane 7) and insulin (10 µg/ml) (lane 8) enhanced C/EBPβ mRNA expression in 3T3-L1 cells, respectively. Although FBS moderately enhanced the expression of C/EBPβ mRNA, its efficacy was lower as compared with MIX, DEX and insulin (lane 9). Prolactin-dependent C/EBPδ expression was not observed.

NIH-3T3 cells incubated in the presence of 10% CS (calf serum) without the above mentioned inducers showed moderate expression of C/EBPβ after 3 hours of incubation (Figure 1B, lane 1). When cells were incubated with different amounts of prolactin, a concentration-dependent increase in the C/EBPβ transcription products was observed. The transcription of C/EBPβ reached saturation with a prolactin concentration of 333 ng/ml (Figure 1B, lane 4). The C/EBPβ mRNA level raised by MIX was about the same as that by prolactin (lane 6). DEX and insulin also enhanced C/EBPβ transcription (lanes 7 and 8). FBS did not induce C/EBPβ mRNA so much as prolactin did (lane 9). These results reveal that prolactin induces the transcription of C/EBPβ, at least in multipotential mesenchymal stem cells.

### [Example 2] Prolactin induces PPARγ transcription in the NIH-3T3 cells and 3T3-L1 preadipocytes:

In multipotential mesenchymal stem cells expressing the C/EBPβ ectopically, PPARγ expression is known to be activated when the C/EBPβ level exceeds a certain threshold (Z. Wu et al. (1995) Genes Dev. 9: 2350-2363). Thus, the inventors of the present invention studied whether the addition of prolactin would induce PPARγ expression.

Different amounts of prolactin were added to the DMEM containing 10% calf serum, insulin (10 µg/ml), DEX (1 µM) and MIX (0.5 mM), and incubated with confluent cells for 48 hours. As the control, the DMEM containing 10% FBS, insulin, DEX and MIX was reacted with confluent cells for a same period. Since the expression of PPARγ mRNA depends on the presence of MIX, DEX and insulin (Z. Wu et al. (1996) Mol Cell Biol. 16:4128-4136), the experiment was conducted in the presence of these three adipocyte differentiation-inducing hormones (adipogenic hormones). Since FBS contains prolactin (R. Biswas and B.K. Vinderhaar (1987) Cancer Res. 47:3509-3514), as the control to eliminate the effects of prolactin, another experiment was also conducted with 10% CS-containing medium supplemented with the above adipogenic hormones.

The total RNA was isolated from the 3T3-L1 cells and NIH-3T3 cells, and Northern blot analysis was performed using the PPARγ cRNA (antisense RNA) as the probe. The results are shown in Figure 2. It was revealed that PPARγ mRNA was induced, depending on the concentration of the prolactin. In spite of the absence of prolactin in the medium for 2 days, low level expression of PPARγ mRNA was observed in the 3T3-L1 cells (Figure 2A, lane 1). When prolactin was added to the medium, the amount of PPARγ mRNA increased significantly (lanes 2 to 5). The medium containing 10% FBS induced PPARγ mRNA as much as the medium containing 333 ng/ml prolactin (compare lanes 4 and 6). In NIH-3T3 cells as well, PPARγ mRNA was induced depending on the amount of prolactin (Figure 2B, lanes 1 to 5). However, in the absence of prolactin, PPARγ mRNA was below the detection level (lane 1). Likewise, with the 3T3-L1 cells, 10% FBS exhibited the ability to induce PPARγ mRNA similarly to that with 333 ng/ml prolactin (compare lanes 4 and 6). Thus, prolactin was proved to induce PPARγ mRNA in these cell lines.

### [Example 3] Addition of prolactin to the differentiation medium stimulates adipocyte differentiation program (adipogenic program) in NIH-3T3 cells:

The above results suggested the possibility to differentiate multipotential mesenchymal stem cells into adipocytes by adding prolactin to the differentiation medium. Thus, using NIH-3T3 cells, differentiation experiments were performed under normal permissive conditions throughout the differentiation process, in the presence of prolactin.

That is, cells were cultured to confluence, then the medium was replaced with the DMEM containing 10% FBS, 1 µM DEX, 0.5 mM MIX, and 10 µg/ml insulin (Day 0). After 48 hours, the medium was replaced with the DMEM containing 10% FBS and 2.5 µg/ml insulin, and thereafter the medium was replaced every two days. This was defined as a normal permissive medium. In addition, 1 µg/ml prolactin was added to two kinds of medium and was incubated in the same manner.

Northern blot analysis was performed in the same manner as described above, using the cRNA probes for aP2 and glycerol-3-phosphate dehydrogenase (GPD) cRNA, which are differentiation marker genes of adipocytes.

With these media, the differentiation marker gene expression, such as aP2 and GPD, were not detected by the Northern analysis even after 12 days of incubation. Further, neither morphologic changes of the cells nor lipid droplets in the cells were observed. These results suggested that mere addition of prolactin to the normal permissive medium is not enough to activate adipogenic program.

Unlike the 3T3-L1 preadipocytes, NIH-3T3 cells are not readily differentiated into adipocytes with normal permissive medium. Thus, a strong permissive media was tested to differentiate these cells into adipocytes.

Compounds known as thiazolidinediones are potent activation factors of PPARγ and strongly promote differentiation into adipocytes (B.M. Forman et al. (1995) Cell 83:803-812; J.M. Lehmann et al. (1995) J. Biol. Chem. 270:12953-12956). The troglitazone is one example of such compounds. It enhances the ability to differentiate multipotential mesenchymal stem cells into adipocytes (J.M. Lehmann et al. (1995) J. Biol. Chem. 270: 12953-12956). Thus, further experiments were conducted using this compound.

First, the cells were incubated in the DMEM containing 10% FBS, 1 µM DEX, 0.5 mM MIX and 10 µg/ml insulin for 48 hours. Then, the medium was replaced with the DMEM containing insulin (2.5 µg/ml) and 10% FBS, as well as 5 µM troglitazone (strong permissive medium). The medium was replaced every two days. In addition, 1 µg/ml prolactin was added to the medium, either before or after the addition of troglitazone, and these cultures were also cultivated in the same manner.

Figure 3 shows the time course of different mRNA expression during the differentiation induction process obtained by Northern blot analysis. The C/EBPβ mRNA level reached a maximum within 24 hours, and the level was maintained up to 48 hours (Figure 3, lanes 2 and 3). After 3 days, the expression level gradually declined (Figure 3, lanes 4 to 7). These characteristics were consistent with those observed for the 3T3-L1 cells (Z. Cao et al. (1991) Genes Dev. 5:1538-1552). Up to the 2nd day, the C/EBPβ mRNA level did not show prolactin-dependent changes (compare lanes 2, 3, 8 and 9). However, after the 3rd day, the level of C/EBPβ mRNA was maintained at a rather higher level in the presence of prolactin (compare lanes 4 to 7, and 10 to 13).

As a result of the Northern blot analysis performed in the same manner as described above, using the C/EBPδ cRNA as a probe, the C/EBPδ mRNA level also increased along with the induction of differentiation into adipocytes, and was maintained a high level for 2 days (Figure 3, lanes 2 and 3). However, the C/EBPδ mRNA level declined sharply on Day 3 and then became almost undetectable. This is in contrast to results obtained with the 3T3-L1 cells. In the 3T3-L1 cells, the C/EBPδ mRNA level was maintained at a high level for 4 days, and then gradually declined (Z. Cao et al. (1991) Genes Dev. 5:1538-1552). No apparent differences in the C/EBPδ mRNA level by the presence or absence of prolactin was detected (compare Figure 3, lanes 2 to 3, and 8 to 13).

It is known that the PPARγ mRNA level rises with differentiation of the cells in the 3T3-L1 cells (Tontonoz, (1994) Cell 79:1147-1156). The inventors of the present invention studied whether the same phenomenon occurs in the NIH-3T3 cells. The PPARγ mRNA level rose from the second day of incubation with MIX, DEX and insulin, and then sharply declined in the absence of prolactin (lanes 1 to 7). In contrast, when prolactin was added to the medium, although the transcription product level was much lower than in the case of 3T3-L1 cells, PPARγ mRNA expression increased dramatically on the second day and was maintained until the 10th day (lanes 3, 9, and 8 to 13).

Since C/EBPβ was induced by prolactin, effects of prolactin on another transcription factor in the C/EBP family, namely C/EBPα mRNA, was investigated. By the Northern blot analysis under the same conditions as described above, using C/EBPα cRNA (antisense RNA) as a probe, C/EBPα mRNA was not detected. This is consistent with the observations in multipotential mesenchymal stem cells, which ectopically express the C/EBPβ (Z. Wu et al. (1995) Genes Dev. 9:2350-2363))).

The Northern blot analysis of the GPD gene and aP2 gene revealed that the addition of troglitazone to prolactin-containing medium induced expression of these two genes (Figure 3). Even in the absence of prolactin, aP2 mRNA was observed from the 3rd day to the 10th day (lanes 4 to 7). Addition of prolactin to these potent permissive media enhanced the expression of the aP2 gene (compare lanes 4 to 7 and 10 to 13 of Figure 3). The effect of prolactin on the GPD gene was even greater. In the absence of prolactin, no GPD mRNA was detected. However, in the presence of prolactin, GPD mRNA appeared on day 8 and continued to increase until day 10(Figure 3, lanes 12 and 13). After 8 days from induction of differentiation, in the presence of prolactin and troglitazone, some of the cells became round shape and showed lipid droplet accumulation; however, the number of cells that accumlate fat droplet was small.

### [Example 4] Effects of prolactin on the adipocyte differentiation program of NIH-3T3 cells that ectopically express the prolactin receptor:

### 4-1)

Whether the prolactin receptor has the ability to convert NIH-3T3 cells into adipocytes was investigated. The rat prolactin receptor gene (PR) (Shirota M et al., (1990) Mol. Endocrinol., 4:1136-1143; provided by Dr. Roland Ball, Friedrich Meischer Institute, Basel, Switzerland) was inserted into pME18S (Mol. Cell. Biol. 8:466-472(1988)), and the plasmid was stably transfected into NIH-3T3 cells together with pSV2neo (Clontech) coding the neomycin-resistance gene by using "lipofectAMINE PLUS (GIBCO BRL)", according to the published instruction. Specifically, the method was as follows: 8 µg of the PR-containing plasmid and 0.4 µg pSV2neo were added in with 20 µl PLUS reagent (GIBCO BRL) and 30 µl lipofectAMINE (GIBCO BRL) into 1.5 ml OPTI-MEM I (GIBCO BRL), incubated at the room temperature for 30 minutes, and then supplemented with 8 ml OPTI-MEM I. Cells seeded the day before in 10 cm dishes at a density of 8 X 10⁵ were rinsed with OPTI-MEM I and supplemented with the above described DNA-containing mixture. After incubating at 37°C for 3 hours, the cells were incubated in the DMEM containing 10% normal calf serum for 24 hours, and then the cells were selected with 0.4 mg/ml G418. As the control, cells transfected with the neomycin-resistance gene (pSV2neo) alone were also selected. To select the transfectants, cells were incubated for 14 days in the presence of G418 to allow the resistant clones to form colonies. Dishes containing about the same number of G418-resistant colonies (60 to 70/dish) were exposed to the above-mentioned high permissive conditions, containing 1 µg/ml prolactin, and were allowed to differentiate in situ for 10 days. Specifically, cells were first incubated for 48 hours in the DMEM containing 10% FBS, 1 µM DEX, 0.5 mM MIX, 10 µg/ml insulin, and 1 µg/ml prolactin. Then the medium was changed with the DMEM containing insulin (2.5 µg/ml), 10% FBS, 5 µM troglitazone and 1 µg/ml prolactin (strong permissive medium) and the cells were allowed to differentiate in situ for 8 days. The media was replaced every two days.

Then, the colonies were fixed with PBS containing 2% formaldehyde and 0.2% glutaraldehyde, and were stained with Oil-Red-O to detect differentiation into adipocytes (accumulation of lipid droplets in cytoplasm) (A. Preece (1972) "Manual for histologic technicians", Boston, MA.: Little, Brown, and Co.). Differentiated cells containing lipid droplets were not observed before the hormone stimulation. However, after incubation in the strong permissive medium for 10 days, colonies of cells, finally differentiated into adipocytes (rounded-shaped cell clusters), were identified by the Oil-Red-O staining (a colony was considered to be differentiated when 50% or more cells of the colony were stained). Colonies without morphological changes, i.e., fibrous colonies, were never stained with Oil-Red-O.

The same experiment was repeated three times. In these medium conditions, approximately 11% of G-418-resistant colonies co-transfected with the prolactin receptor gene finally differentiated into adipocytes. Approximately 2% of the colonies of the control cell, which was transfected only with the neomycin-resistant gene, differentiated. The results are summarized in Table 1. In the table, "total colony " indicates total numbers of G418-resitant colonies observed in three independent transfection experiments. The "differentiated colony" indicates the numbers of colonies wherein 50% or more cells were stained by Oil-Red-O. In some colonies, adipocytes were dispersed. Such colonies were only slightly stained with Oil-Red-O, and thus were excluded from the differentiated colony counts.

**Table 1**

| in situ colony differentiation assay | | | |
|---|---|---|---|
| Expression plasmid | Total colony | Differentiated colony | Ratio(%) |
| pSV2neo | 232 | 5 | 2.1 |
| PR+pSV2neo | 213 | 24 | 11.3 |

These data confirmed that prolactin receptors played an important role in differentiation into adipocytes.

### 4-2)

In the above experiment of adipocyte differentiation, there were large differences in the differentiation abilities among cell clones. Thus, to eliminate this problem, cells were handled in groups in the following experiments.

Stable transfectants that ectopically express prolactin receptors were isolated by G-418 selection, and more than 20,000 clones were collected. Since the C/EBPβ and PPARγ mRNA were induced by prolactin in the parent cells, prolactin dose-dependency of expression of these genes was studied with the above cells.

Cells cultured to confluence were incubated in the DMEM containing 10% calf serum (CS), with increasing amounts of prolactin, for 3 hours. The total RNA was isolated, and the C/EBPβ mRNA was analysed by the Northern blot analysis.

In the control cells that were transfected with the neomycin-resistant gene alone, dose-dependent expression of the C/EBPβ mRNA similar to that of the parent cells was observed (Figures 1 and 4A, lanes 1 to 5). In the prolactin receptor-expressing cell group, sensitivity to prolactin was further enhanced (Figure 4A, lanes 6 to 10).

Then, the effect of prolactin on PPAR γ mRNA was also examined. Cells were cultured in the DMEM containing 10% calf serum (CS), 10 µg/ml insulin, 1 µM DEX and 0.5 mM MIX, with increasing amounts of prolactin, for 48 hours. Figure 4B shows the results of the Northern blot analyses of the RNA prepared from the control cells and prolactin receptor-expressing cells. The control cells showed a prolactin dose-dependent expression of PPARγ mRNA, similar to that of the parent cells (Figures 2B and 4B, lanes 1 to 5). The sensitivity of prolactin to PPARγ mRNA expression of the cell group, prepared so as to express prolactin receptors ectopically, was highly enhanced (compare lanes 1 to 5 and 6 to 10).

Since strong ectopic expression of the prolactin receptors promotes induction of the expression of PPARγ mRNA and C/EBPβ mRNA, the differentiation processes of the prolactin receptor-expressing cells and control cells were analyzed in the presence and absence of prolactin. Specifically, the medium was first changed with the DMEM containing 10% FBS, 1 µM DEX, 0.5 mM MIX and 10 µg/ml insulin (Day 0) and then incubated for 48 hours, changing the medium with the DMEM containing insulin (2.5 µg/ml), 10% FBS, as well as 5 µM troglitazone (strong permissive medium). The medium was replaced every two days. In addition, 1 µg/ml prolactin was added to the medium before and after adding troglitazone, and these cultures were also incubated in the same manner.

The entire differentiation program of these transfectants was analyzed by the Northern blot analyses of adipocyte-specific marker genes mentioned below (Figure 5). In the control group, C/EBPβ mRNA expression slightly increased after 3 days in the presence of prolactin, (Figure 5A), as in the case of the parent cells (Figure 3). In the prolactin receptor-expressing group, in the presence of prolactin, the amount of C/EBPβ mRNA rose remarkably on Day 3 (compare lanes 4 and 9 of Figures 5A and 5B), and then a slightly higher expression level was maintained. For the C/EBPδ mRNA, no apparent effect by the addition of prolactin was observed in both groups during the entire differentiation process. In the control group, in the absence of prolactin, PPARγ mRNA expression reached a peak on Day 2 and then sharply decreased (lanes 1 to 6). The expression of the PPARγ was enhanced as the prolactin receptor was expressed ectopically. PPARγ expression was maintained relatively high, especially in cells expressing the prolactin receptor with prolactin during the late phase of the differentiation program (panels A and B, lanes 9 to 11). In both cells, aP2 mRNA became detectable by Day 3 (lane 4). Expression of aP2 mRNA was strongly activated by the addition of prolactin (compare panels A and B, lanes 9 to 11). In the prolactin receptor-expressing cells, aP2 mRNA was expressed much stronger by the addition of prolactin. In the control cells, while almost no GPD mRNA was detected even after 8 days in the absence of prolactin (panel A, lane 6), GPD mRNA was detected on Day 8 in the presence of prolactin (lane 11). Similarly, while GPD mRNA was not detected in the absence of prolactin (panel B, lanes 1 to 6), addition of prolactin enhanced GPD mRNA induction in the prolactin receptor-expressing cells; GPD mRNA became detectable on Day 5 and was increased further on Day 8 (Figure 5B, lanes 10 and 11). Expression of C/EBP α, another marker gene related to the adipocyte differentiation, was not detected throughout the 8-day cultivation period.

Subsequently, the differentiation of cells were observed microscopically. As in 4-1), the prolactin receptor-expressing cells and control cells were incubated in the adipocyte-induction medium for 10 days, and then were stained with Oil-Red-O. Little differentiation was observed in the control cells to which only the neomycin-resistant gene had been transferred (in the absence of prolactin) (1% or less, Figure 6, Neo and Table 2). When prolactin was added to these cells, the number of round-shaped cells increased. It was estimated that 4% of the cells had differentiated, according to the results of staining (Figure 6 Neo+prolactin and Table 2). On the other hand, when the prolactin receptor-expressing cells were induced for differentiation in the same manner, approximately 18% of the cells differentiated into adipocytes and accumulated lipid droplets in the presence of prolactin (Figure 6, Neo+PR+prolactin and Table 2). The differentiation rate of this cell group was lower in the absence of prolactin as well (5%, Figure 6 Neo+PR, Table 2). This finding indicates that effective differentiation of NIH-3T3 cells into adipocytes requires not only strong expression of the prolactin receptor but also their activation. Upon repeating these stable transfection experiments four times, 13 to 23% of the G-418 resistant cells, cotransfected with the prolactin receptor gene, converted into adipocytes with lipid accumulation. The results are shown in Table 2. In the table, the "differentiation rate" refers to counts (mean ± SD) in 200 cells in 10 random microscopic fields of each four independent transfection experiments.

**Table 2**

| Differentiation assay of pooled clones | |
|---|---|
| Expression plasmid | Differentiation rate(%) |
| pSV2neo | <1 |
| pSV2neo+prolactin | 4 ± 1 |
| PR+pSV2neo | 5 ± 2 |
| PR+pSV2neo+prolactin | 18 ± 5 |

Thus, taking all the above results into account, it was verified that the prolactin receptor play an important role in differntiation of multipotential mesenchymal stem cells into adipocytes.

### Industrial applicability

It was revealed by the present invention that signals by prolactin and PPARγ act in coordination to induce differentiation of multipotential mesenchymal stem cells into adipocytes. The screening for compounds that promote or inhibit differentiation into adipocytes is enabled by utilizing this differentiation system of adipocytes or by targeting factors related to adipocyte differentiation signal transduction. The use as a drug of compounds obtained by the screening is anticipated, for prevention and/or treatment of diseases related with adipocyte differentiation, such as insulin-resistant diabetes,.

In addition, since agonists of PPARγ suppress production of inflammatory cytokines by monocytes (C.Jiang et al. (1988) Nature 39:82-86), prolactin and its agonists, which increase PPARγ mRNA, have the potential to act together with PPARγ agonists as a therapeutic agents for rheumatoid arthritis.

## Claims

1. A method for differentiating a multipotential mesenchymal stem cell into adipocytes, comprising incubating the multipotential mesenchymal stem cell in the presence of prolactin or substance with an equivalent effect to prolactin.

2. The method of claim 1, wherein said incubation is conducted in the presence of a PPARγ activator.

3. The method of claims 1 or 2, wherein the multipotential mesenchymal stem cell expresses the exogenous prolactin receptor.

4. The method of any of claim 1 to 3, wherein the multipotential mesenchymal stem cell is the NIH-3T3 cell.

5. A method of screening for inhibitors or accelerators of adipocyte differentiation, which comprises the steps of:
(a) incubating multipotential mesenchymal stem cells in the presence of (i) a test compound and (ii) prolactin or a substance with an equivalent effect to prolactin;
(b) detecting the differentiation of said cells into adipocytes;
(c) selecting the test compound which inhibits or promotes the differentiation by comparing the result of (b) with that under the absence of the test compound (control).

6. The method of claim 5, wherein a PPARγ activator is further added.

7. The method of claim 5 or 6, wherein the multipotential mesenchymal stem cells express the exogenous prolactin receptor.

8. The method of any of claim 5 to 7, wherein adipocyte differentiation is detected using as an indicator (a) fat accumulation in the cytoplasm, (b) expression of adipocyte differentiation-inducing genes, or (c) expression of adipocyte marker genes as an indicator.

9. The method of any of claim 5 to 8, wherein the multipotential mesenchymal stem cells are NIH-3T3 cells.

10. A method of screening for inhibitors or accelerators of adipocyte differentiation, which comprises the steps of:
(a) contacting prolactin with a test compound; and
(b) selecting the test compound that binds to prolactin.

11. A method for screening inhibitors or accelerators of adipocyte differentiation, which comprises the steps of:
(a) contacting a prolactin receptor and a test compound; and
(b) selecting the test compound that binds to the prolactin receptor.

12. A method of screening for inhibitors or accelerators of adipocyte differentiation, which comprises the steps of:
(a) contacting prolactin with the prolactin receptor in the presence of a test compound; and
(b) selecting the test compound that inhibits or promotes binding of prolactin to the prolactin receptor.

13. A method of screening for inhibitors or accelerators of adipocyte differentiation, which comprises the steps of:
(a) providing cells expressing the endogenous prolactin receptor and transfected with a vector containing a promoter, which is activated in response to prolactin, and a reporter gene functionally fused downstream to it;
(b) contacting the cells with(i) a test compound or (ii) a test compound with prolactin to said cell; and
(c) detecting the activity of the reporter gene in said cell.

14. An adipocyte differentiation inhibitor, which contains a prolactin inhibitor as the active ingredient.

15. An adipocyte differentiation inhibitor, which contains a prolactin receptor inhibitor as the active ingredient.

16. An adipocyte differentiation accelerator, which contains a prolactin activator as the active ingredient.

17. An adipocyte differentiation accelerator, which contains a prolactin receptor activator as the active ingredient.

18. An adipocyte differentiation inhibitor or accelerator, which can be isolated by the methods of claims 5 to 13.

19. An adipocyte differentiation accelerator, which contains prolactin as the active ingredient.

20. A PPARγ expression-inducing agent, which contains prolactin as the active ingredient.

21. A C/EBPβ expression-inducing agent, which contains prolactin as the active ingredient.

22. A compound that inhibits or promote adipocyte differentiation, which inhibits or accelerates intracellular signal transduction of prolactin.


SEQUENCE LISTING

<110> Helix Research Institute

<120> Methods for inducing adipocyte differentiation,
      and chemicals which control adipocyte
      differentiation and methods for screening them.

<130> H1-007PCT

<140>
<141>

<150> JP   1999-24625
<151> 1999-02-02

<160> 6

<210> 1
<211> 30
<212> DNA
<213> Artificial Sequence

<220>

<223> Description of Artificial Sequence: an
      artificially synthesized primer sequence

<400> 1
atggagtcgg ccgacttcta cgaggcggag                                        30

<210> 2
<211> 30
<212> DNA
<213> Artificial Sequence

<220>
<223> Description of Artificial Sequence: an
      artificially synthesized primer sequence

<400> 2
atgcaccgcc tgctggcctg ggacgcagca                                        30

<210> 3
<211> 30
<212> DNA
<213> Artificial Sequence

<220>
<223> Description of Artificial Sequence: an
      artificially synthesized primer sequence

<400> 3

atgagcgccg cgcttttcag cctggacagc 30

<210> 4

<211> 30

<212> DNA

<213> Artificial Sequence

<220>

<223> Description of Artificial Sequence: an

artificially synthesized primer sequence

<400> 4

atggttgaca cagagatgcc attctggccc 30

<210> 5

<211> 30

<212> DNA

<213> Artificial Sequence

<220>

<223> Description of Artificial Sequence: an

artificially synthesized primer sequence

<400> 5

atgtgtgatg cctttgtggg aacctggaag 30

<210> 6

<211> 30

<212> DNA

<213> Artificial Sequence

<220>

<223> Description of Artificial Sequence: an

artificially synthesized primer sequence

<400> 6

atggcgtttc aaaaggcagt gaaggggact 30